# FASCICULE DE BREVET EUROPEEN

(11) **EP 1 289 383 B1**
(45) Date de publication et mention de la délivrance du brevet: **03.05.2006**
(21) Numéro de dépôt: 01945214.3
(22) Date de dépôt: 29.05.2001
(51) Int. Cl.: A23L 1/30, A23L 1/275, A23L 1/305, A61K 8/00, A61Q 19/00

(54) **COMPOSITION PRIMAIRE CONTENANT UN COMPOSE BIOACTIF LIPOPHILE**
PRIMÄRZUSAMMENSETZUNG ENTHALTEND EINEN LIPOPHILEN, BIOAKTIVEN STOFF
PRIMARY COMPOSITION CONTAINING A LIPOPHILIC BIOACTIVE COMPOUND

(30) Priorité: 30.05.2000 EP 00111542
(43) Date de publication de la demande: 12.03.2003
(73) Titulaire: SOCIETE DES PRODUITS NESTLE S.A., 1800 Vevey (CH)
(72) Inventeur: BORTLIK, Karlheinz, CH-1073 Savigny (CH); SAUCY, Françoise, CH-1807 Blonay (CH); DURUZ, Eliane, CH-1066 Epalinges (CH); RICHELLE, Myriam, CH-1073 Savigny (CH); LAMBELET, Pierre, CH-1806 Saint-Légier (CH); BAUR, Markus, 89081 ULM (DE); PFEIFER, Andrea, M., A., CH-1806 St-Légier (CH)
(74) Mandataire: Chautard, Cécile
(86) Numéro de dépôt international: PCT/EP2001/006145
(87) Numéro de publication internationale: WO 2001/091588

(56) Documents cités:
- EP-A- 0 278 284
- EP-A- 0 986 963
- GB-A- 1 521 691
- US-A- 4 522 743
- US-A- 5 601 760

## Description

La présente invention concerne une composition primaire comprenant un composé bioactif lipophile, ainsi qu'une composition orale contenant ladite composition primaire et son procédé de préparation.

On connaît déjà des compositions disponibles sur le marché contenant un composé bioactif lipophile, à savoir du lycopène. Le lycopène est un produit naturel, qui est connu pour avoir de multiples fonctionnalités, notamment celle d'antioxydant. Le lycopène est présent dans différents produits naturels, notamment la tomate, le melon, la goyave et le pamplemousse. La composition généralement disponible sur le marché et contenant du lycopène est une oléorésine. Le problème avec cette oléorésine est qu'on a constaté que le lycopène qu'elle contenait n'était pas suffisamment biodisponible. Le but de la présente invention est de disposer d'un produit contenant du lycopène et ayant une meilleure biodisponibilité que les produits sur le marché à l'heure actuelle.

Le brevet EP 278284 concerne une composition pulvérulente contenant un caroténoïde de synthèse. Le problème avec cette composition est qu'elle n'est pas utilisable dans le domaine alimentaire et d'autre part elle est envisagée dans un but de coloration.

US 5,601,760 décrit un agent de microencapsulation à base de protéines de petit-lait et une méthode pour microencapsuler des matériaux volatiles ou non volatiles pour augmenter leur stabilité.

GB 1 521 691 décrit un procédé de préparation d'une emulsion d' oléorésine dans lequel on fait une dispersion ou une solution d'oléolrésine dans un acide avec une huile essentielle. On mélange et on émulsifie la solution avec une solution aqueuse colloïdale en présence de protéine de petit-lait.

La présente invention concerne une composition primaire comprenant un composé bioactif lipophile et une protéine de petit lait. Par composé bioactif, on entend un composé ayant un effet bénéfique sur le métabolisme humain ou animal. Le but de la présente invention est de mettre à disposition du consommateur une composition obtenue à partir de produits naturels. Comme il sera mentionné ci-dessous, on vise aussi selon l'invention une protection du composé bioactif lipophile.

Le composé bioactif lipophile est obtenu, extrait, enrichi ou purifié à partir de plante, de microorganisme, de levure ou d'un produit d'origine animale. Par obtenu, on entend que le produit bioactif est directement disponible dans le commerce. Par extrait, on entend qu'on a extrait le principe bioactif. Par enrichi, on entend qu'on a séparé le plus possible les composés non bioactifs. Par purifié, on entend qu'on récupère uniquement le composé bioactif.

Dans le cas d'un composé bioactif provenant d'une plante, celle-ci est choisie dans le groupe constitué par la tomate (tomate entière, extrait de tomate, chair de tomate,purée de tomate, pelures de tomate avec ou sans les pépins), le soja, le thé vert, les grains de café vert, les épices (gingembre et autres épices), les grains de raisin, le cacao et les céréales. Le microorganisme peut être tout type de bactérie qui produit un composé bioactif lipophile. Par exemple, on peut envisager comme microorganisme un probiotique, notamment une bactérie d'acide lactique. La levure peut être toute levure qui produit un composé bioactif lipophile, par exemple un Saccharomyces. Le produit d'origine animale est choisi dans le groupe constitué par un extrait de foie et une fraction de lait. Par fraction de lait, on entend toute partie du lait.

Dans la composition primaire selon l'invention, le composé bioactif lipophile est choisi dans le groupe constitué par les caroténoïdes, les polyphénols, les vitamines lipophiles, les flavonoïdes, les isoflavones, les curcuminoïdes, les céramides, les proanthocyanidines, les terpénoïdes, les stérols, les phytostérols, les stérol-esters, les tocotriénols, le squalène, les rétinoïdes, seuls ou en mélange. Les caroténoïdes sont présent notamment dans la tomate, la carotte, la pêche jaune, l'abricot et l'orange. Le lycopène est un caroténoïde qui est particulièrment privilégié dans la présente composition. Les polyphénols sont présents notamment dans le thé vert, le café, le cacao, le vin rouge. Les vitamines lipophiles sont présentes notamment dans de nombreux légumes. Les flavonoïdes et le isoflavones sont présents notamment dans le soja, le thé, les oignons, le vin. Les curcuminoïdes sont présents notamment dans le gingembre. Les céramides sont des glycolipides présents notamment dans les dérivés de levure et les dérivés d'origine animale. Les proanthocyanidines sont des flavonoïdes présents notamment dans les grains de raisin. Les terpénoïdes sont présents dans les épices. Les stérols, phytostérols et stérols-esters sont présents notamment dans les huiles végétales, les graines , les noix et les légumes. Les tocotriénols sont présents notamment dans le son de riz, l'orge, le blé, l'huile de palme, le seigle et l'avoine. Le squalène est présent notamment dans le foie des poissons, l'huile d'olive, l'huile de germes de blé, l'huile de son de riz. Finalement les rétinoïdes sont présents notamment dans le foie, le jaune d'oeuf et les produits laitiers.

Dans une forme de réalisation préférée de la composition primaire selon l'invention, le composé bioactif lipophile est obtenu à partir de la tomate, par exemple de la purée de tomate ou un extrait de tomate. La présence de lycopène dans la tomate est un avantage pour la présente invention. Le composé bioactif peut aussi être un extrait de soja. Il est aussi possible d'avoir un mélange d'extrait de tomate et de soja.

La composition selon l'invention peut se présenter sous la forme de poudre, de liquide ou de gel.

Comme déjà mentionné ci-dessus, le but premier de la présente invention est d'avoir une composition contenant un composé bioactif lipophile ayant une meilleure biodisponibilité que le composé seul et le but second est de mettre à disposition du consommateur une composition bien hydrodispersible, si on choisit la forme de poudre, en l'occurence une poudre dispersible dans l'eau à température ambiante. Un troisième but est de protéger le composé bioactif lipophile par la protéine du petit lait.

Dans la composition selon l'invention, la protéine est la protéine du petit-lait, par exemple de l'isolat de protéine de petit lait. Par protéine de petit lait, on entend un produit contenant au moins 80 % de protéines de petit lait.

La composition primaire selon l'invention peut en outre contenir de la vitamine E et de la vitamine C. La vitamine E (tocophérol) peut être d'origine exogène ou endogène. La vitamine C est ajoutée dans la composition.

La composition contient en outre des émulsifiants, des stabilisants et des additifs. Comme émulsifiants, on utilise les émulsifiants compatibles dans le domaine alimentaire, comme les phospholipides, par exemple la lécithine, le polyoxyéthylène sorbitane mono- ou tristéarate, monolaurate, monopalmitate, mono- ou trioleate, un mono- ou diglycéride. Comme stabilisant, on utilise tout type de stabilisant connu dans l'alimentation, dans la cosmétique ou la pharmacie. Comme additifs, on utilise les arômes, les colorants, et tout autre additif connu dans l'alimentation, la cosmétique ou la pharmacie. Ces émulsifiants, stabilisants et additifs sont ajoutés en fonction de l'utilisation finale de ladite composition primaire.

La composition selon l'invention contient jusqu'à 50 % de composé bioactif lipophile et jusqu'à 90 % de protéine de petit lait.

Dans une forme de réalisation préférée de la composition primaire selon l'invention, celle-ci contient de l'oléorésine de tomate, de l'extrait de soja et de la protéine de petit lait. Par oléorésine de tomate dans la présente description, on entend un extrait lipidique de ladite plante incluant des caroténoïdes, comme le lycopène, des triglycérides, des phospholipides, du tocophérol et d'autres composés plus mineurs. Par extrait de soja, on entend un extrait de soja contenant une forte teneur en isoflavone. On peut aussi envisager d'autres plantes contenant des caroténoïdes, en particulier le melon, la goyave, le pamplemousse, l'abricot, le cynorrhodon, la carotte, la pêche et l'orange.

La présente invention concerne en outre une composition orale comprenant la composition primaire décrite ci-dessus dans un produit alimentaire, dans un complément alimentaire, dans une préparation cosmétique ou dans une préparation pharmaceutique.

Cette composition ingérable par voie orale permet d'augmenter la biodisponibilité du composé bioactif lipophile dans le corps et retarder le vieillisssement de la peau. Comme produit alimentaire complémenté par la composition primaire ci-dessus on peut citer les yogurts, les boissons liquides, le chocolat, les crèmes glacées, les céréales, les poudres de chocolat, le café, les produits culinaires comme la mayonnaise, la purée de tomate ou les sauces à salade, les produits de nutrition infantile, les produits de nutrition entérale ou les aliments pour animaux familiers. Dans ce cas, la poudre est dissoute dans les aliments ou boissons mentionnés ci-dessus de manière à avoir une prise journalière comprise entre 0.001 et 50 mg de composé bioactif lipophile, par exemple comme le lycopène. De préférence, on envisage une prise journalière de l'ordre de 5 à 20 mg par jour.

On peut également prévoir selon l'invention un complément alimentaire sous forme de dragées, de pilules, de gélules, de sirop, de gel, de crème ou de tablettes dosées de 0,001 à 100 % de ladite composition primaire, qu'on peut alors prendre directement avec de l'eau ou par tout autre moyen connu. Ce complément comprend en outre un édulcorant, un stabilisant, un additif, un arôme et un colorant.

La composition orale peut également être une préparation cosmétique contenant la composition primaire et un composé actif pour la peau connu par l'homme du métier.

La composition orale peut également être une préparation pharmaceutique contenant la composition primaire et un composé pharmaceutique, par exemple en application topique ou ingérable par voie orale.

L'invention concerne également une composition cosmétique contenant la composition primaire décrite ci-dessus. Dans ce cas, la teneur en composition primaire est comprise entre 10⁻¹⁰ et 10 %. De préférence, la composition cosmétique contient entre 10⁻⁸ et 5 % de composé bioactif lipophile.

Cette composition utilisable par la voie topique contient en outre une graisse ou une huile utilisable en cosmétique, par exemple celles mentionné dans l'ouvrage CTFA, Cosmetic Ingredients Handbook, Washington. On peut également ajouter d'autres ingrédients cosmétiquement actifs. La composition contient en outre un agent structurant et un émulsifiant.

On peut également ajouter à la composition d'autres excipients, colorants, parfums ou opacifiants.

La présente invention concerne en outre l'utilisation de la composition orale ou de la composition cosmétique décrite ci-dessus pour protéger les tissus de la peau contre le vieillissement, en particulier pour inhiber la dégradation de la peau et/ou des muqueuses par l'inhibition des collagénases et l'augmentation de la synthèse du collagène.

La présente invention concerne en outre le procédé de préparation de la composition primaire décrite ci-dessus, dans lequel la protéine de petit lait est mélangée avec le composé bioactif lipophile.

Dans une première forme de réalisation du procédé selon l'invention,
- on dissout la protéine de petit lait dans de l'eau,
- on dissout le composé bioactif lipophile dans un solvant choisi parmi l'acétone, l'éthanol et l'isopropanol,
- on mélange les deux solutions,
- on évapore le solvant et
- on obtient une dispersion.

Dans une première variante du procédé selon l'invention, on obtient une dispersion. Dans une seconde variante, on traite thermiquement la dispersion pour obtenir un gel. Et dans une troisième variante, on fait un séchage de la dispersion par pulvérisation ou par lyophilisation pour obtenir une poudre. La composition selon l'invention peut être directement utilisable telle quelle ou en mélange, comme il sera explicité ci-dessous.

On dissout la protéine de petit lait dans l'eau à une température voisine ou légèrement supérieure à la température ambiante. On utilise une oléorésine qui contient entre 1 et 40 % de lycopène. Les quantités sont données en poids/poids. Quand on dissout l'oléorésine dans le solvant, on travaille avec un rapport de ladite oléorésine sur le solvant de l'ordre de 1:1 à 1:900 en poids.

Le solvant est choisi parmi l'acétone, l'éthanol ou l'isopropanol. Lorsqu'on mélange la phase aqueuse avec le solvant , on choisit un rapport solvant/eau en volume de l'ordre de 60/40.

Après le mélange des deux phases, on laisse reposer le mélange pendant 30 à 60 min à une température un peu plus élevée que la température ambiante, par exemple de l'ordre de 30 °C et on commence par chasser le solvant sous un vide modéré. Par vide modéré, on entend un vide compris entre 200 et 300 mbar. Si on veut une poudre,on élimine l'eau soit sous vide, soit par pulvérisation, soit par lyophilisation. Par vide, on entend un vide compris entre 40 et 50 mbar. Si on veut un gel, on chauffe l'émulsion ou on opère selon toute autre technique connue de l'homme de métier pour faire ledit gel.

Dans une seconde forme de réalisation du procédé selon l'invention,
- on mélange le composé bioactif lipophile avec un solvant choisi parmi l'acétone, l'éthanol et l'isopropanol,
- on mélange la composition obtenue avec la poudre de protéine de petit lait et
- on évapore le solvant pour obtenir une composition en poudre.

Le solvant utilisé est le même que celui mentionné ci-dessus.

La suite de la description est faite maintenant en référence aux exemples.

### Exemple 1 : préparation de la composition sous forme de poudre

On dissout 13,3 kg d'isolat de protéines de petit-lait dans 330 L d'eau déminéralisée et on agite le mélange pendant 6 heures à 25-30 °C. Séparément, on mélange 550 g d'oléorésine de la Société Lycored contenant 6 % de lycopène dans 438 L d'acétone et on agite le mélange. On mélange ensuite les 2 solutions pendant 60 min à 30 °C. Le mélange final est chauffé modérément et on chasse l'acétone à une pression modérée. Finalement, on chasse partiellement l'eau à une pression de 40-50 mbar. On obtient une solution aqueuse de 200 kg d'isolat de protéine de petit-lait et d'oléorésine.

Cette solution est ensuite séchée par pulvérisation.

A partir de cette poudre, des essais ont été effectués avec des personnes auxquelles on a donné journalièrement cette poudre contenant 25 mg de lycopène et d'autres caroténoïdes contenues dans l'oléorésine et 12,5 g de protéines de petit lait : la prise a été faite par dissolution de la poudre dans du jus de pomme. Par rapport à la référence, qui était la purée de tomate contenant la même quantité de lycopène, on a constaté une biodisponibilité du lycopène à partir de la poudre selon l'invention égale à celle à partir de la purée de tomate : cette étude a été effectuée sur une durée de 8 semaines. Il faut rappeler que la purée de tomate est considérée pour l'homme de métier comme le produit ayant la meilleure biodisponibilité du lycopène. Cette mise en évidence a été faite par un dosage du lycopène dans le plasma du sang.

### Exemple 2 : Préparation de dragées

On fait une dispersion de 550 g d'oléorésine de l'exemple 1 avec un émulsifiant dans de l'éthanol. On mélange cette dispersion avec 1100 g de protéine de petit lait et 1100 g d'extrait de soja (contenant 40 % d'isoflavone). On chasse le solvant pour obtenir une poudre.

La poudre ainsi obtenue est mélangée avec de l'acide ascorbique, ainsi que d'autres additifs, tels que un ou des édulcorants , des épaississants ainsi que des additifs alimentaires permettant la préparation sous forme de dragées. On dragéifie ensuite le mélange obtenu.

On prépare ainsi des dragées de l'ordre de 700 mg contenant 33 mg de lycopène, 70 mg d'extrait de soja, 70 mg de protéine de petit lait, 40 mg d'acide ascorbique, le reste étant des édulcorants, des épaississants et des additifs alimentaires pour arriver à 700 mg.

### Exemple 3 : Composition cosmétique

On prépare un lait pour le visage contenant 7 % d'huile de vaseline, 2 % de poudre selon l'exemple 1, 3 % de monostéarate de glycéryle, stéarate de polyéthylène-glycol, 0,4 % de polymère carboxyvinylique, 0,7 % d'alcool stéarylique, 3 % de protéines de soja, 0,4 % de NaOH, un conservateur et le complément à 100 est de l'eau.

### Exemple 4 : Composition cosmétique

On prépare un gel pour le visage contenant 10 % de glycérine, 2 % de poudre selon l'exemple 1, 1 % de cocoamphodiacétate de disodium, un conservateur et le complément à 100 est de l'eau.

### Example 5 : Etude de la stabilité du lycopène

Il est connu que la lumière et l'oxygène causent une dégradation du lycopène. On a effectué une analyse en phase aqueuse de la stabilité du lycopène seul et du lycopène en association avec la protéine de petit lait selon l'invention. Au bout d'une journée en phase aqueuse pour le lycopène seul, il ne reste que 40 % de lycopène, alors qu'avec le petit lait , il en reste pratiquement 90 %. Au bout de deux jours, avec le petit lait il en reste 60 %, alors que le lycopène est presque totalement dégradé s'il est seul.

On a donc bien un effet protecteur du lycopène par la protéine de petit lait.

## Revendications

1. Composition primaire sous la forme d'une poudre, comprenant au moins un composé bioactif lipophile, et une protéine de petit-lait, susceptible d'être obtenue en
- mélangeant le composé bioactif lipophile avec un solvant choisi parmi l'acétone, l'éthanol et l'isopropanol,
- mélangeant la composition obtenue avec la poudre de protéine de petit-lait et
- évaporant le solvant pour obtenir ladite composition sous forme de poudre,
le composé bioactif lipophile ayant une biodisponibilité augmentée par rapport au composé seul.

2. Composition primaire sous forme de liquide ou de gel, comprenant au moins un composé bioactif lipophile, et une protéine de petit-lait, susceptible d'être obtenue en
- dissolvant la protéine de petit-lait dans de l'eau,
- dissolvant le composé bioactif lipophile dans un solvant choisi parmi l'acétone, l'éthanol et l'isopropanol,
- mélangeant les deux solutions,
- évaporant le solvant pour obtenir ladite composition sous forme de dispersion,
le composé bioactif lipophile ayant une biodisponibilité augmentée par rapport au composé seul.

3. Composition primaire selon la revendication 1 ou 2, **caractérisée en ce que** le composé bioactif lipophile est obtenu, extrait, enrichi ou purifié à partir de plante, de microorganisme, de levure ou d'un produit d'origine animale.

4. Composition primaire selon la revendication 1 ou 2, **caractérisée en ce que** la plante est choisie dans le groupe constitué par la tomate, le soja, le thé vert, les grains de café vert, les épices, les grains de raisin, le cacao, le gingembre et les céréales.

5. Composition primaire selon la revendication 1 ou 2, **caractérisée en ce que** le microorganisme est tout type de bactérie qui produit un composé bioactif lipophile.

6. Composition selon la revendication 1 ou 2, **caractérisée en ce que** la levure est une levure qui produit un composé bioactif lipophile.

7. Composition selon la revendication 1 ou 2, **caractérisée en ce que** le produit d'origine animale est choisi dans le groupe constitué par un extrait de foie et une fraction de lait.

8. Composition selon l'une des revendications 1 à 7, **caractérisée en ce que** le composé bioactif lipophile est choisi dans le groupe constitué par les caroténoïdes, les polyphénols, les vitamines lipophiles, les flavonoïdes, les isoflavones, les curcuminoïdes, les céramides, les proanthocyanidines, les terpénoïdes, les stérols, les phytostérols, les stérol-esters, les tocotriénols, le squalène, les rétinoïdes, seul ou en mélange.

9. Composition primaire selon l'une des revendications 1 à 7, **caractérisée en ce que** le composé bioactif lipophile est un extrait de tomate, un extrait de soja ou un mélange d'extrait de tomate et de soja.

10. Composition selon l'une des revendications 1 à 9, **caractérisée en ce qu'**elle contient en outre de la vitamine C et/ou du tocophérol.

11. Composition selon l'une des revendications 1 à 10, **caractérisée en ce qu'**elle contient en outre des émulsifiants, des stabilisants et des additifs.

12. Composition selon l'une des revendications 1 à 11, **caractérisée en ce qu'**elle contient jusqu'à 50 % de composé bioactif lipophile et jusqu'à 90 % de protéine de petit lait.

13. Composition selon la revendication 12, **caractérisée en ce qu'**elle comprend de l'oléorésine de tomate, de l'extrait de soja et de la protéine de petit lait.

14. Composition orale comprenant la composition primaire selon l'une des revendications 1 à 13 dans un produit alimentaire, dans un complément alimentaire, dans une préparation cosmétique ou dans une préparation pharmaceutique.

15. Composition orale selon la revendication 14, **caractérisée en ce que** le produit alimentaire est choisi dans le groupe constitué par un yoghurt, une boisson liquide, un chocolat, une crème glacée, des céréales, de la poudre de chocolat, du café, l'alimentation animale.

16. Composition orale selon la revendication 14, **caractérisée en ce que** le complément alimentaire comprend en outre un édulcorant, un stabilisant, un arôme et un colorant et se présente sous forme de dragées, pillules, gélules, sirop, gel ou crème.

17. Composition orale selon la revendication 14, **caractérisée en ce que** la préparation cosmétique comprend en outre un composé actif pour la peau.

18. Composition orale selon l'une des revendications 14 à 17, **caractérisée en ce que** la teneur de la composition primaire est comprise entre 0,001 et 100 %.

19. Composition orale selon la revendication 18, **caractérisée en ce que** la teneur de la composition primaire est comprise entre 10 et 50 %.

20. Composition cosmétique comprenant la composition primaire selon l'une des revendications 1 à 13.

21. Composition cosmétique selon la revendication 20, **caractérisée en ce que** la teneur en composition primaire est comprise entre 10⁻¹⁰ % et 10 %.

22. Utilisation de la composition orale ou de la composition cosmétique selon l'une des revendications 14 à 21 pour protéger les tissus de la peau contre le vieillissement.

23. Procédé pour la préparation d'une composition primaire sous forme de liquide ou de gel, comprenant au moins un composé bioactif lipophile, et une protéine de petit-lait, le composé bioactif lipophile ayant une biodisponibilité augmentée par rapport au composé seul, **caractérisé en ce que**
- on dissout la protéine de petit lait dans de l'eau,
- on dissout le composé bioactif lipophile dans un solvant choisi parmi l'acétone, l'éthanol et l'isopropanol,
- on mélange les deux solutions,
- on évapore le solvant et
- on obtient une dispersion.

24. Procédé pour la préparation d'une composition primaire sous la forme d'une poudre, comprenant au moins un composé bioactif lipophile, et une protéine de petit-lait, le composé bioactif lipophile ayant une biodisponibilité augmentée par rapport au composé seul, **caractérisé en ce que**
- on mélange le composé bioactif lipophile avec un solvant choisi parmi l'acétone, l'éthanol et l'isopropanol
- on mélange la composition obtenue avec la poudre de protéine de petit lait et
- on évapore le solvant pour obtenir une composition en poudre.

## Claims

1. Primary composition in the form of a powder, which comprises at least one lipophilic bioactive compound and a whey protein and is capable of being obtained by
- mixing the lipophilic bioactive compound with a solvent selected from acetone, ethanol and isopropanol,
- mixing the resulting composition with the whey protein powder and
- evaporating off the solvent to obtain said composition in powder form,
the lipophilic bioactive compound having increased bioavailability compared with the compound alone.

2. Primary compound in liquid or gel form, which comprises at least one lipophilic bioactive compound and a whey protein and is capable of being obtained by
- dissolving the whey protein in water,
- dissolving the lipophilic bioactive compound in a solvent selected from acetone, ethanol and isopropanol,
- mixing the two solutions,
- evaporating off the solvent to obtain said composition in the form of a dispersion,
the lipophilic bioactive compound having increased bioavailability compared with the compound alone.

3. Primary composition according to claim 1 or 2, **characterised in that** the lipophilic bioactive compound is obtained, extracted, enriched or purified starting from a plant, from a microorganism, from a yeast or from a product of animal origin.

4. Primary composition according to claim 1 or 2, **characterised in that** the plant is selected from the group consisting of tomato, soybean, green tea, unroasted coffee beans, spices, grapes, cocoa, ginger and cereals.

5. Primary composition according to claim 1 or 2, **characterised in that** the microorganism is any type of bacterium that produces a lipophilic bioactive compound.

6. Composition according to claim 1 or 2, **characterised in that** the yeast is a yeast that produces a lipophilic bioactive compound.

7. Composition according to claim 1 or 2, **characterised in that** the product of animal origin is selected from the group consisting of a liver extract and a milk fraction.

8. Composition according to any one of claims 1 to 7, **characterised in that** the lipophilic bioactive compound is selected from the group consisting of carotenoids, polyphenols, lipophilic vitamins, flavonoids, isoflavones, curcuminoids, ceramides, proanthocyanidins, terpenoids, sterols, phytosterols, sterol esters, tocotrienols, squalene, retinoids, alone or in a mixture.

9. Primary composition according to any one of claims 1 to 7, **characterised in that** the lipophilic bioactive compound is a tomato extract, a soybean extract or a mixture of tomato extract and soybean extract.

10. Composition according to any one of claims 1 to 9, **characterised in that** it further comprises vitamin C and/or tocopherol.

11. Composition according to any one of claims 1 to 10, **characterised in that** it further comprises emulsifiers, stabilisers and additives.

12. Composition according to any one of claims 1 to 11, **characterised in that** it comprises up to 50 % lipophilic bioactive compound and up to 90 % whey protein.

13. Composition according to claim 12, **characterised in that** it comprises tomato oleoresin, soybean extract and whey protein.

14. Oral composition comprising the primary composition according to any one of claims 1 to 13 in a food product, in a food supplement, in a cosmetic preparation or in a pharmaceutical preparation.

15. Oral composition according to claim 14, **characterised in that** the food product is selected from the group consisting of a yoghurt, a liquid drink, chocolate, an ice-cream, cereals, chocolate powder, coffee, animal food.

16. Oral composition according to claim 14, **characterised in that** the food supplement further comprises a sweetener, a stabiliser, a flavouring and a colouring and is in the form of dragées, pills, gelatin capsules, syrup, gel or cream.

17. Oral composition according to claim 14, **characterised in that** the cosmetic preparation further comprises a compound that is active in respect of the skin.

18. Oral composition according to any one of claims 14 to 17, **characterised in that** the content of the primary composition is from 0.001 to 100 %.

19. Oral composition according to claim 18, **characterised in that** the content of the primary composition is from 10 to 50 %.

20. Cosmetic composition comprising the primary composition according to any one of claims 1 to 13.

21. Cosmetic composition according to claim 20, **characterised in that** the content of primary composition is from 10⁻¹⁰ % to 10 %.

22. Use of the oral composition or of the cosmetic composition according to any one of claims 14 to 21 for protecting skin tissue against ageing.

23. Process for the preparation of a primary composition in liquid or gel form, comprising at least one lipophilic bioactive compound and a whey protein, the lipophilic bioactive compound having increased bioavailability compared with the compound alone, **characterised in that**
- the whey protein is dissolved in water,
- the lipophilic bioactive compound is dissolved in a solvent selected from acetone, ethanol and isopropanol,
- the two solutions are mixed,
- the solvent is evaporated off and
- a dispersion is obtained.

24. Process for the preparation of a primary composition in the form of a powder, comprising at least one lipophilic bioactive compound and a whey protein, the lipophilic bioactive compound having increased bioavailability compared with the compound alone, **characterised in that**
- the lipophilic bioactive compound is mixed with a solvent selected from acetone, ethanol and isopropanol,
- the resulting composition is mixed with the whey protein powder and
- the solvent is evaporated off to obtain a powdered composition.

## Patentansprüche

1. Grundzusammensetzung in Form von Pulver, umfassend mindestens eine bioaktive lipophile Verbindung und ein Molkeprotein, die herstellbar ist, indem man
- die lipophile bioaktive Verbindung mit einem Lösungsmittel, das aus Aceton, Ethanol und Isopropanol ausgewählt ist, mischt,
- die erhaltene Zusammensetzung mit dem Molkeproteinpulver mischt,
- das Lösungsmittel abdampft, um die Zusammensetzung in Form von Pulver zu erhalten,
wobei die bioaktive lipophile Verbindung gegenüber der Verbindung allein eine erhöhte Bioverfügbarkeit besitzt.

2. Grundzusammensetzung in Form von Flüssigkeit oder Gel, umfassend mindestens eine bioaktive lipophile Verbindung und ein Molkeprotein, die erhältlich ist, indem man
- das Molkeprotein in Wasser löst,
- die bioaktive lipophile Verbindung in einem Lösungsmittel, das aus Aceton, Ethanol und Isopropanol ausgewählt ist, löst,
- die beiden Lösungen mischt,
- das Lösungsmittel abdampft, um die Zusammensetzung in Form einer Dispersion zu erhalten,
wobei die bioaktive lipophile Verbindung gegenüber der Verbindung allein eine erhöhte Bioverfügbarkeit besitzt.

3. Grundzusammensetzung nach Anspruch 1 oder 2, **dadurch gekennzeichnet, dass** die bioaktive lipophile Verbindung aus Pflanzen, Mikroorganismen, Hefe oder einem Produkt tierischen Ursprungs erhalten, extrahiert, angereichert oder gereinigt ist.

4. Grundzusammensetzung nach Anspruch 1 oder 2, **dadurch gekennzeichnet, dass** die Pflanze aus der Gruppe ausgewählt ist, die aus Tomate, Soja, grünem Tee, grünen Kaffeebohnen, Gewürzen, Weintraubenkernen, Kakao, Ingwer und Getreide besteht.

5. Grundzusammensetzung nach Anspruch 1 oder 2, **dadurch gekennzeichnet, dass** der Mikroorganismus jeder Bakterientyp ist, der eine bioaktive lipophile Verbindung erzeugt.

6. Zusammensetzung nach Anspruch 1 oder 2, **dadurch gekennzeichnet, dass** die Hefe eine Hefe ist, die eine bioaktive lipophile Verbindung erzeugt.

7. Zusammensetzung nach Anspruch 1 oder 2, **dadurch gekennzeichnet, dass** das Produkt tierischen Ursprungs aus der Gruppe ausgewählt ist, die aus einem Leberextrakt und einer Milchfraktion besteht.

8. Zusammensetzung nach einem der Ansprüche 1 bis 7, **dadurch gekennzeichnet, dass** die bioaktive lipophile Verbindung aus der Gruppe ausgewählt ist, die aus Carotenoiden, Polyphenolen, lipophilen Vitaminen, Flavonoiden, Isoflavonen, Curcuminoiden, Ceramiden, Proanthocyanidinen, Terpenoiden, Sterolen, Phytosterolen, Sterolestern, Tocotrienolen, Squalen, Retinoiden allein oder in Mischung besteht.

9. Grundzusammensetzung nach einem der Ansprüche 1 bis 7, **dadurch gekennzeichnet, dass** die bioaktive lipophile Verbindung ein Tomatenextrakt, ein Sojaextrakt oder eine Tomaten- und Sojaextraktmischung ist.

10. Zusammensetzung nach einem der Ansprüche 1 bis 9, **dadurch gekennzeichnet, dass** sie außerdem Vitamin C und/oder Tocopherol enthält.

11. Zusammensetzung nach einem der Ansprüche 1 bis 10, **dadurch gekennzeichnet, dass** sie außerdem Emulgatoren, Stabilisatoren und Zusätze enthält.

12. Zusammensetzung nach einem der Ansprüche 1 bis 11, **dadurch gekennzeichnet, dass** sie bis 50 % bioaktive lipophile Verbindung und bis 90 % Molkeprotein enthält.

13. Zusammensetzung nach Anspruch 12, **dadurch gekennzeichnet, dass** sie Tomaten-Oleoresin, Sojaextrakt und Molkeprotein enthält.

14. Orale Zusammensetzung, umfassend die Grundzusammensetzung nach einem der Ansprüche 1 bis 13 in einem Nahrungsmittelprodukt, in einem Nahrungsmittelzusatzstoff, in einer kosmetischen Zubereitung oder in einer pharmazeutischen Zubereitung.

15. Orale Zusammensetzung nach Anspruch 14, **dadurch gekennzeichnet, dass** das Nahrungsmittelprodukt aus der Gruppe ausgewählt ist, die aus Joghurt, einem flüssigen Getränk, Schokolade, Eiscreme, Getreide, Schokoladepulver, Kaffee, Tiernahrung besteht.

16. Orale Zusammensetzung nach Anspruch 14, **dadurch gekennzeichnet, dass** der Nahrungsmittelzusatzstoff außerdem ein Süßungsmittel, einen Stabilisator, ein Aroma und einen Farbstoff enthält und in Form von Dragees, Pillen, Gelatinekapseln, Sirup, Gel oder Creme vorliegt.

17. Orale Zusammensetzung nach Anspruch 14, **dadurch gekennzeichnet, dass** die kosmetische Zubereitung außerdem eine aktive Verbindung für die Haut umfasst.

18. Orale Zusammensetzung nach einem der Ansprüche 14 bis 17, **dadurch gekennzeichnet, dass** der Gehalt an Grundzusammensetzung 0,001 bis 100 % beträgt.

19. Orale Zusammensetzung nach Anspruch 18, **dadurch gekennzeichnet, dass** der Gehalt an Grundzusammensetzung 10 bis 50 % beträgt.

20. Kosmetische Zusammensetzung, umfassend die Grundzusammensetzung nach einem der Ansprüche 1 bis 13.

21. Kosmetische Zusammensetzung nach Anspruch 20, **dadurch gekennzeichnet, dass** der Gehalt an Grundzusammensetzung 10⁻¹⁰ bis 10 % beträgt.

22. Verwendung der oralen Zusammensetzung oder der kosmetischen Zusammensetzung nach einem der Ansprüche 14 bis 21 zum Schutz der Gewebe der Haut gegen das Altern.

23. Verfahren zur Herstellung einer Grundzusammensetzung in Form von Flüssigkeit oder Gel, umfassend mindestens eine bioaktive lipophile Verbindung und ein Molkeprotein, wobei die bioaktive lipophile Verbindung gegenüber der Verbindung allein eine erhöhte Bioverfügbarkeit besitzt, **dadurch gekennzeichnet, dass** man
- das Molkeprotein in Wasser löst,
- die bioaktive lipophile Verbindung in einem Lösungsmittel, das aus Aceton, Ethanol und Isopropanol ausgewählt ist, löst,
- die beiden Lösungen mischt,
- das Lösungsmittel abdampft und
- eine Dispersion erhält.

24. Verfahren zur Herstellung einer Grundzusammensetzung in Form eines Pulvers, umfassend mindestens eine bioaktive lipophile Verbindung und ein Molkeprotein, wobei die bioaktive lipophile Verbindung gegenüber der Verbindung allein eine erhöhte Bioverfügbarkeit besitzt, **dadurch gekennzeichnet, dass** man
- die bioaktive lipophile Verbindung mit einem Lösungsmittel, das aus Aceton, Ethanol und Isopropanol ausgewählt ist, mischt,
- die erhaltene Zusammensetzung mit dem Molkeproteinpulver mischt und
- das Lösungsmittel abdampft, um eine pulverförmige Zusammensetzung zu erhalten.
